# EUROPEAN PATENT SPECIFICATION

(11) **EP 1 398 316 B1**
(45) Date of publication and mention of the grant of the patent: **14.06.2006**
(21) Application number: 03255795.1
(22) Date of filing: 16.09.2003
(51) Int. Cl.: C07D 491/04

(54) **Process for the preparation of 1,3-Dihydro-6-methylfuro(3,4-c)pyridin-7-ol Derivatives**
Verfahren zur Herstellung 1,3-Dihydro-6-methylfuro(3,4-c)pyridin-7-ol-derivate
Procédé de préparation de dérivés de 1,3-Dihydro-6-methylfuro(3,4-c)pyridin-7-ol

(30) Priority: 16.09.2002 GB 0221494
(43) Date of publication of application: 17.03.2004
(73) Proprietor: Generics (UK) Limited, Potters Bar, Herts EN6 1AG (GB)
(72) Inventor: Gore, Vinayak G. Merck Development Centre, 410 208 Maharashtr (IN); Ghadge, Manoj M. Merck Development Centre, 410 208 Maharashtr (IN); Gupta, Ashwinikumar K. Merck Development Centre, 410 208 Maharashtr (IN)
(74) Representative: Elend, Almut Susanne

(56) References cited:
- DE-A- 4 010 657
- GB-A- 2 234 244
- US-A- 4 383 998
- US-A- 4 585 776
- US-A- 5 130 252
- BRESLOW R ET AL: "ARTIFICIAL TRANSAMINASES LINKING PYRIDOXAMINE TO BINDING CAVITIES: CONTROLLING THE GEOMETRY" JOURNAL OF THE AMERICAN CHEMICAL SOCIETY, AMERICAN CHEMICAL SOCIETY, WASHINGTON, DC, US, vol. 112, no. 13, 1990, pages 5212-5219, XP002036255 ISSN: 0002-7863

## Description

### Technical field

The present invention relates to a process for the preparation of 1,3-dihydro-6-methylfuro[3,4-c]pyridin-7-ol derivatives 5 and salts thereof. The numbering of derivatives 5 is indicated below.

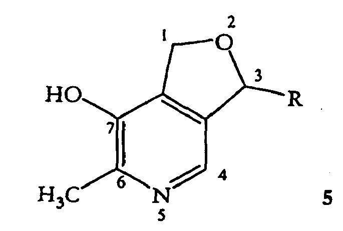

### Background art

Certain 1,3-dihydro-6-methylfuro[3,4-c]pyridin-7-ol derivatives 5 are known to have antihypertensive and diuretic properties and are therefore potentially useful as pharmaceuticals. One such compound, the hydrochloride salt of 3-(4-chlorophenyl)-1,3-dihydro-6-methylfuro[3,4-c]pyridin-7-ol (cicletanine hydrochloride 5b) is marketed as a pharmaceutical for the treatment of hypertension. 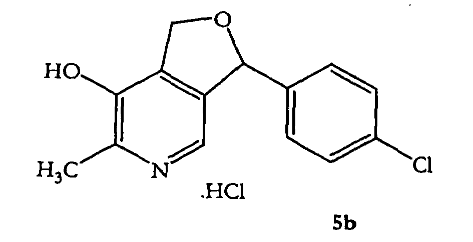 Processes for the preparation of 1,3-dihydro-6-methylfuro[3,4-c]pyridin-7-ol derivatives 5 have been disclosed in patents US 4,383,998 and US 5,043,448. Procedures to this class of compounds have also been disclosed by P.D. Sattsangi and C.J. Argoudelis in J. Org. Chem., 33(4), 1337-1341 (1968) and by W. Korytnyk, E.J. Kris and P.R. Singh in J. Org. Chem., 29, 574-579 (1964).

However, known processes for the preparation of 1,3-dihydro-6-methylfuro[3,4-c]pyridin-7-ol derivatives 5 are not satisfactory, particularly for industrial scale manufacture, as they involve the use of reagents such as chromium trioxide and manganese dioxide. These reagents are not acceptable for manufacturing activities because of the stringent limitations on residual metal content present in the effluent of chemical processes. The use of these reagents is also inconvenient, because they require an elaborate and difficult work up procedure to isolate any product obtained.

The present inventors have developed a short, simple process for the preparation of 1,3-dihydro-6-methylfuro[3,4-c]pyridin-7-ol derivatives 5 and salts thereof that avoids the use of such disadvantageous reagents. The process is easy to scale up for industrial scale manufacturing, and all raw materials and chemicals used in the process are environmentally friendly and do not require special treatment for disposal.

### Summary of the invention

The present invention provides a process for the preparation of a 1,3-dihydro-6-methylfuro[3,4-c]pyridin-7-ol derivative 5 or a salt thereof, comprising the steps of:
(a) protecting the 3-hydroxy and 4-hydroxymethyl substituents of pyridoxine 1 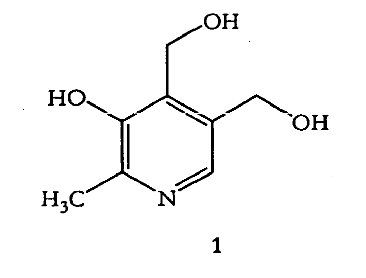 or a salt thereof, preferably pyridoxine hydrochloride 1a, to yield a 3,4-protected pyridoxine 2 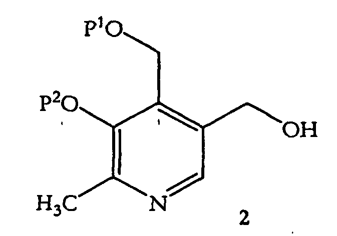 or a salt thereof, wherein P¹ and P² are independently protecting groups or together form one protecting group,
(b) oxidising the 5-hydroxymethyl substituent of the 3,4-protected pyridoxine 2 or the salt thereof, using aqueous sodium hypochlorite in the presence of a catalytic amount of TEMPO (2,2,6,6-tetramethyl-1-piperidinyloxy) to yield a 3,4-protected pyridoxal 3 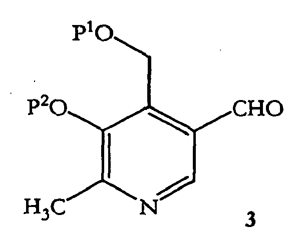 or a salt thereof,
(c) adding a nucleophile to the aldehyde of the 3,4-protected pyridoxal 3 or the salt thereof to yield an adduct 4 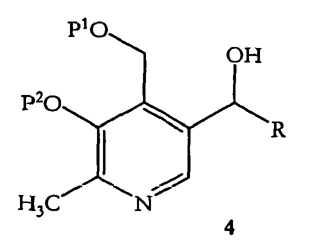 or a salt thereof, wherein R is any atom or group, preferably an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, and
(d) deprotecting the adduct 4 or the salt thereof, and cyclo-dehydrating the 4- and 5-hydroxymethyl substituents of the deprotected adduct 4 or the salt thereof to yield the 1,3-dihydro-6-methylfuro[3,4-c]pyridin-7-ol derivative 5 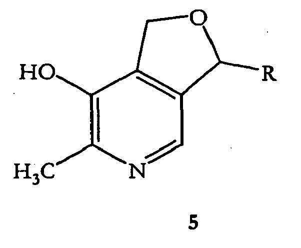 or a salt thereof.

For the purposes of the present invention, an "alkyl" group is defined as a monovalent saturated hydrocarbon, which may be straight-chained or branched, or be or include cyclic groups. Examples of alkyl groups are methyl, ethyl, *n*-propyl, *i-*propyl, *n*-butyl, *i*-butyl, *t*-butyl and *n*-pentyl groups. Preferably an alkyl group is straight-chained or branched and does not include any heteroatoms in its carbon skeleton. Preferably an alkyl group is a C₁-C₁₂ alkyl group, which is defined as an alkyl group containing from 1 to 12 carbon atoms. More preferably an alkyl group is a C₁-C₆ alkyl group, which is defined as an alkyl group containing from 1 to 6 carbon atoms. An "alkylene" group is similarly defined as a divalent alkyl group.

An "alkenyl" group is defined as a monovalent hydrocarbon, which comprises at least one carbon-carbon double bond, which may be straight-chained or branched, or be or include cyclic groups. Examples of alkenyl groups are vinyl, allyl, but-1-enyl and but-2-enyl groups. Preferably an alkenyl group is straight-chained or branched and does not include any heteroatoms in its carbon skeleton. Preferably an alkenyl group is a C₂-C₁₂ alkenyl group, which is defined as an alkenyl group containing from 2 to 12 carbon atoms. More preferably an alkenyl group is a C₂-C₆ alkenyl group, which is defined as an alkenyl group containing from 2 to 6 carbon atoms. An "alkenylene" group is similarly defined as a divalent alkenyl group.

An "alkynyl" group is defined as a monovalent hydrocarbon, which comprises at least one carbon-carbon triple bond, which may be straight-chained or branched, or be or include cyclic groups. Examples of alkynyl groups are ethynyl, propargyl, but-1-ynyl and but-2-ynyl groups. Preferably an alkynyl group is straight-chained or branched and does not include any heteroatoms in its carbon skeleton. Preferably an alkynyl group is a C₂-C₁₂ alkynyl' group, which is defined as an alkynyl group containing from 2 to 12 carbon atoms. More preferably an alkynyl group is a C₂-C₆ alkynyl group, which is defined as an alkynyl group containing from 2 to 6 carbon atoms. An "alkynylene" group is similarly defined as a divalent alkynyl group.

An "aryl" group is defined as a monovalent aromatic hydrocarbon. Examples of aryl groups are phenyl, naphthyl, anthracenyl and phenanthrenyl groups. Preferably an aryl group does not include any heteroatoms in its carbon skeleton. Preferably an aryl group is a C₄-C₁₄ aryl group, which is defined as an aryl group containing from 4 to 14 carbon atoms. More preferably an aryl group is a C₆-C₁₀ aryl group, which is defined as an aryl group containing from 6 to 10 carbon atoms. An "arylene" group is similarly defined as a divalent aryl group.

Where a combination of groups is referred to as one moiety, for example, arylalkyl, arylalkenyl, arylalkynyl, alkylaryl, alkenylaryl or alkynylaryl, the last mentioned group contains the atom by which the moiety is attached to the rest of the molecule. A typical example of an arylalkyl group is benzyl.

A "cycloheteroalkyl", "cycloheteroalkenyl" and "heteroaryl" group is defined as a cyclic alkyl, alkenyl or aryl group respectively, which comprises at least one heteroatom N, O or S as part of the cyclic system.

For the purposes of this invention, an optionally substituted alkyl, alkenyl, alkynyl, aryl, arylalkyl, arylalkenyl, arylalkynyl, alkylaryl, alkenylaryl or alkynylaryl group may be substituted with one or more of -F, -Cl, -Br, -I, -CF₃, -CCl₃, -CBr₃, -CI₃, -OH, -SH, -NH₂, -CN, -NO₂,-COOH, -R¹-O-R², -R¹-S-R², -R¹-SO-R², -R¹-SO₂-R², -R¹-SO₂-OR², -R¹O-SO₂-R², -R¹-SO₂-N(R²)₂, -R¹-NR²-SO₂-R², -R¹O-SO₂-OR², -R¹O-SO₂-N(R²)₂, -R-NR²-SO₂-OR², -R¹-NR²-SO₂-N(R²)₂, -R¹-N(R²)₂, -R¹-N(R²)₃⁺, -R¹-P(R²)₂, -R¹-Si(R²)₃, -R¹-CO-R², -R¹-CO-OR², -R¹O-CO-R², -R¹-CO-N(R²)₂, -R¹-NR²-CO-R²,-R¹O-CO-OR², -R¹O-CO-N(R²)₂, -R¹-NR²-CO-OR², -R¹-NR²-CO-N(R²)₂, -R¹-CS-R², -R¹-CS-OR², -R¹O-CS-R², -R¹-CS-N(R²)₂, -R¹-NR²-CS-R², -R¹O-CS-OR², -R¹O-CS-N(R²)₂, -R¹-NR²-CS-OR², -R¹-NR²-CS-N(R²)₂ or -R². In this context, -R¹- is independently a chemical bond, a C₁-C₁₀ alkylene, C₁-C₁₀ alkenylene or C₁-C₁₀ alkynylene group. -R² is independently hydrogen, unsubstituted C₁-C₆ alkyl or unsubstituted C₆-C₁₀ aryl. Optional substituent(s) are not taken into account when calculating the total number of carbon atoms in the parent group substituted with the optional substituent(s).

Any optional substituent may be protected. Suitable protecting groups for protecting optional substituents are known in the art, for example from "Protective Groups in Organic Synthesis" by T.W. Greene and P.G.M. Wuts (Wiley-Interscience, 2^{nd} edition, 1991).

For the purposes of this invention, a "salt" is any pharmaceutically acceptable acid addition salt, including but not limited to a hydrohalogenic acid salt such as hydrofluoric, hydrochloric, hydrobromic or hydroiodic acid salt; an inorganic acid salt such as nitric, perchloric, sulfuric or phosphoric acid salt; an organic acid salt such as a sulfonic acid salt (for example methanesulfonic, trifluoromethanesulfonic, ethanesulfonic, isethionic, benzenesulfonic, p-toluenesulfonic or camphorsulfonic acid salt), acetic, malic, fumaric, succinic, citric, tartaric, benzoic, gluconic, lactic, mandelic, mucic, pamoic, pantothenic, oxalic or maleic acid salt; and an aminoacid salt such as ornithinic, glutamic or aspartic acid salt. A preferred salt is a hydrohalogenic, sulphuric, phosphoric or organic acid salt. A more preferred salt is a hydrochloric acid salt.

R can be any atom or group. Preferably R is an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton.

Preferably R is an alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, and which may be optionally substituted with one or more of -F, -Cl, -Br, -I, -CF₃, -CCl₃, -CBr₃, -CI₃, -OH, -SH, - NH₂, -CN, -NO₂, -COOH, -R¹-O-R²,-R¹-S-R², -R¹-SO-R², -R¹-SO₂-R², -R¹-N(R²)₂, -R¹-Si(R²)₃, -R¹-CO-R², -R¹-CO-OR², -R¹O-CO-R², -R¹-CO-N(R²)₂, -R¹-NR²-CO-R², - R¹-CS-R² or -R², wherein
- R¹- is independently a chemical bond, a C₁-C₁₀ alkylene, C₁-C₁₀ alkenylene or C₁-C₁₀ alkynylene group, and
- R² is independently hydrogen, unsubstituted C₁-C₆ alkyl or unsubstituted C₆-C₁₀ aryl.

Preferably R is an alkyl, alkenyl, alkynyl, aryl, arylalkyl, arylalkenyl, arylalkynyl, alkylaryl, alkenylaryl or alkynylaryl group, which does not include any heteroatoms in its carbon skeleton, and which may be optionally substituted with one or more of -F, -Cl, -Br, -I, -CF₃, -CCl₃, -CBr₃, -CI₃, -OH, -SH, -NH₂, -CN, -NO₂, -COOH, -OR²,-SR², -SO-R², -SO₂-R², -N(R²)₂, -Si(R²)₃, -CO-R², -CO-OR², -O-CO-R², -CO-N(R²)₂, -NR²-CO-R², -CS-R² or -R², wherein
- R² is independently hydrogen, unsubstituted C₁-C₆ alkyl or unsubstituted C₆-C₁₀ aryl.

Preferably R is an alkyl, an optionally substituted aryl or an optionally substituted heteroaryl group, which does not include any heteroatoms in its carbon skeleton. In the most preferred embodiment, R is 4-chloro-phenyl.

P¹ and P² independently can be any protecting group or together form one protecting group. Suitable protecting groups are commonly known in the art, for example from Chapter 2 of "Protective Groups in Organic Synthesis" by T.W. Greene and P.G.M. Wuts (Wiley-Interscience, 2^{nd} edition, 1991).

Preferably protecting groups P¹ and P² are independently -R³ -Si(R³)₃, -COR³, -CO₂R³, -SO₃⁻ or -SO₃R³, wherein each R³ is independently an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton. Alternatively, protecting groups P¹ and P² together form one protecting group -CHR⁴-, -C(R⁴)₂-, -C(OR⁴)R⁵-, -C(N(R⁴)₂)R⁵-, -Si(R⁴)₂-, -Si(R⁴)₂-O-Si(R⁴)₂-, -CO- or -BR⁴-, wherein each R⁴ and each R⁵ is independently an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, or wherein R⁴ and R⁴, or R⁴ and R⁵, together form an optionally substituted cycloalkyl or cycloheteroalkyl group.

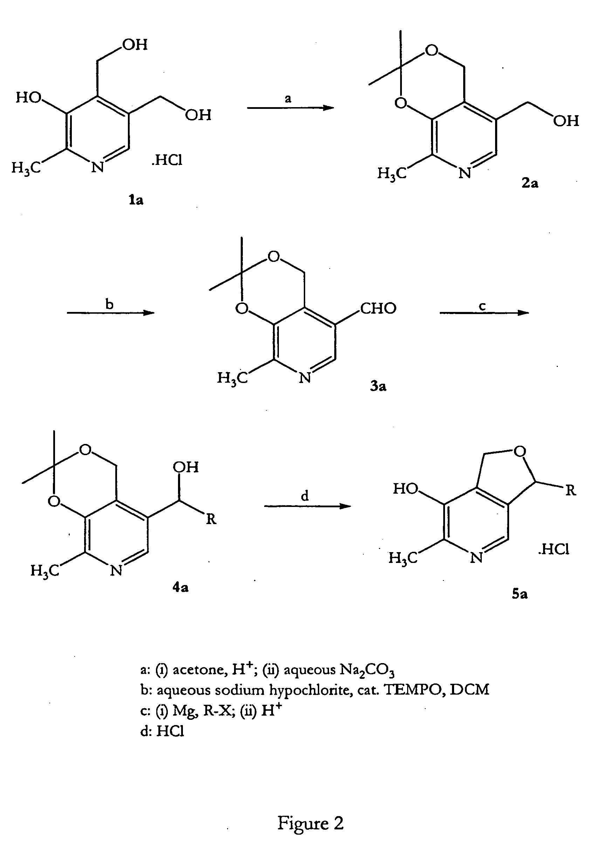

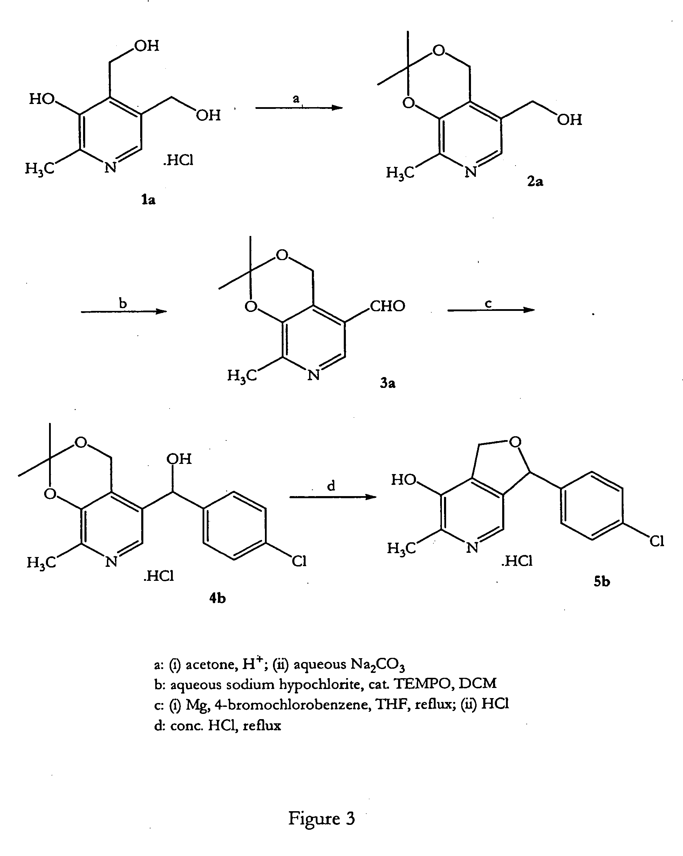

Preferably protecting groups P¹ and P² together form one protecting group. More preferably protecting groups P¹ and P² together form one protecting group -C(R⁴)₂-, wherein each R⁴ is independently an unsubstituted C₁-C₆ alkyl group. In the most preferred embodiment, P¹ and P² together form a ketal -C(R⁴)₂-, with both R⁴ being methyl, forming 3,4-isopropylidine pyridoxine **2a** as shown in Figures 2 and 3.

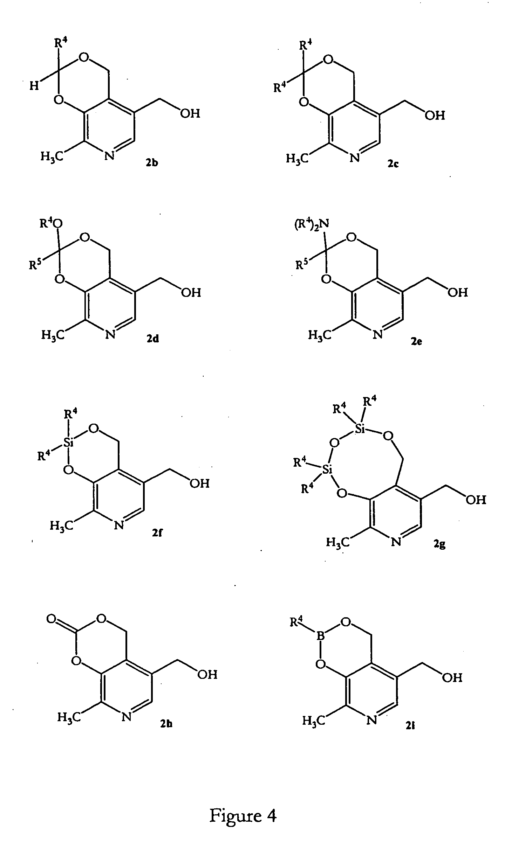

When protecting groups P¹ and P² together form an acetal -CHR⁴-, the 3,4-protected pyridoxine is of the formula **2b** shown in Figure 4, wherein R⁴ is hydrogen, or an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton.

When protecting groups P¹ and P² together form a ketal -C(R⁴)₂-, the 3,4-protected pyridoxine is of the formula **2c** shown in Figure 4, wherein each R⁴ is independently an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, or both R⁴ together form an optionally substituted cycloalkyl or cycloheteroalkyl group.

When protecting groups P¹ and P² together form a cyclic ortho ester -C(OR⁴)R⁵-, the 3,4-protected pyridoxine is of the formula **2d** shown in Figure 4, wherein R⁴ is an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, and R⁵ is hydrogen, or an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, or wherein R⁴ and R⁵ together form an optionally substituted cycloheteroalkyl group.

When protecting groups P¹ and P² together form a cyclic ortho amide -C(N(R⁴)₂)R⁵-, the 3,4-protected pyridoxine is of the formula **2e** shown in Figure 4, wherein each R⁴ is independently an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, and R⁵ is hydrogen, or an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, or wherein both R⁴, or R⁴ and R⁵, together form an optionally substituted cycloheteroalkyl group.

When protecting groups P¹ and P² together form a silyl derivative -Si(R⁴)₂- or -Si(R⁴)₂-O-Si(R⁴)₂-, the 3,4-protected pyridoxine is of the formula **2f** or **2g** shown in Figure 4, wherein each R⁴ is independently an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton.

When protecting groups P¹ and P² together form a cyclic carbonate -CO-, the 3,4-protected pyridoxine is of the formula **2h** shown in Figure 4.

When protecting groups P¹ and P² together form a cyclic borate -BR⁴-, the 3,4-protected pyridoxine is of the formula **2i** shown in Figure 4, wherein R⁴ is an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton.

Preferably the nucleophile used in step (c) is:
(a) a Grignard reagent RMgX, wherein R is any atom or group, preferably an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, and wherein X is a leaving group, preferably a halide, more preferably a chloride, bromide or iodide;
(b) Li-R, wherein R is any atom or group, preferably an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton;
(c) Zn-R, wherein R is an optionally substituted alkyl group;
(d) Na-C≡C-Rⁿ, wherein Rⁿ is any atom or group, preferably an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, and wherein R in formulae **4, 4a, 5** and **5a** is -C≡C-Rⁿ;
(e) R'₂Al-CH=CHRⁿ, wherein Rⁿ is any atom or group, preferably an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, wherein each R' is independently an alkyl group, and wherein R in formulae **4, 4a, 5** and **5a** is -CH=CHRⁿ;
(f) R'₃Sn-CH₂-CH=CHRⁿ, wherein Rⁿ is any atom or group, preferably an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, wherein each R' is independently an alkyl group, and wherein R in formulae **4, 4a, 5** and **5a** is -CH₂-CH=CHRⁿ;
(g) R₂CuLi, wherein R is an unsubstituted alkyl group, preferably an unsubstituted C₁₋₄ alkyl group, more preferably methyl; or
(h) (R'O)₃TiR, (R'₂N)₃TiR or (R'O)₃ZrR, wherein R is any atom or group, preferably an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, and wherein each R' is independently an unsubstituted alkyl group.

Preferably the nucleophile used in step (c) is:
(a) a Grignard reagent RMgX which is prepared *in situ* from magnesium and R-X, wherein R and X are as defined above with reference to Grignard reagent RMgX, preferably wherein R-X is 4-bromochlorobenzene;
(b) Li-R which is prepared *in situ* from lithium and R-X, wherein R is as defined above with reference to Li-R and X is a leaving group, preferably a halide, more preferably a chloride, bromide or iodide; or
(c) (R'O)₃TiR, (R'₂N)₃TiR or (R'O)₃ZrR which is prepared by transmetallation of a Grignard reagent RMgX or Li-R with (R'O)₃TiX, (R'₂N)₃TiX or (R'O)₃ZrX, wherein R is as defined above with reference to (R'O)₃TiR, (R'₂N)₃TiR or (R'O)₃ZrR and X is independently a halide, preferably a chloride, bromide or iodide.

In the most preferred embodiment, the nucleophile used in step (c) is a Grignard reagent RMgX, preferably wherein the addition of the Grignard reagent RMgX to the aldehyde of the 3,4-protected pyridoxal 3 or a salt thereof is carried out at a temperature of 50 to 130°C, preferably 60 to 110°C, most preferably about 70°C.

In the most preferred embodiment, the oxidation of step (b) is carried out in the presence of less than 3 mol% KBr compared to the alcohol 2 to be oxidised, preferably less than 1 mol% KBr, more preferably less than 0.5 mol% KBr, and most preferably substantially in the absence of KBr.

Preferably the deprotection and cyclo-dehydration of step (d) is achieved in one step by heating in HCl, preferably by refluxing in conc. HCl, yielding a 1,3-dihydro-6-methylfuro[3,4-c]pyridin-7-ol derivative hydrochloric acid salt **5a**. Concentrated HCl preferably contains at least 30% HCl, preferably at least 35% HCl, and more preferably at least 37% HCl.

A preferred derivative 5 obtainable by the process of the present invention is 3-(4-chlorophenyl)-1,3-dihydro-6-methylfuro[3,4-c]pyridin-7-ol (cicletanine) and its hydrochloric acid salt **5b**. Preferably the 1,3-dihydro-6-methylfuro[3,4-c]pyridin-7-ol derivative **5** or the salt thereof obtainable by the process of the present invention is suitable for use as an antihypertensive or a diuretic.

The 1,3-dihydro-6-methylfuro[3,4-c]pyridin-7-ol derivatives **5** or salts thereof have at least one chiral centre and can therefore exist in the form of various stereoisomers. The present invention embraces processes for the preparation of all of these stereoisomers and mixtures thereof. Mixtures of these stereoisomers can be resolved by conventional methods, for example, chiral chromatography, fractional recrystallisation, derivatisation to form diastereomers and subsequent resolution, and resolution using enzymes.

The 1,3-dihydro-6-methylfuro[3,4-c]pyridin-7-ol derivative **5** or a salt thereof obtainable by the process of the present invention preferably comprises at least 95% of the (R)- or the (S)-enantiomer, preferably at least 98% of the (R)- or the (S)-enantiomer, and more preferably at least 99% of the (R)- or the (S)-enantiomer.

### Brief description of the drawings

Figure 1 is a schematic illustration of the process of the present invention.
Figures 2 and 3 are schematic illustrations of preferred processes of the invention.
Figure 4 illustrates preferred protecting groups P¹ and P².

### Detailed description of the invention

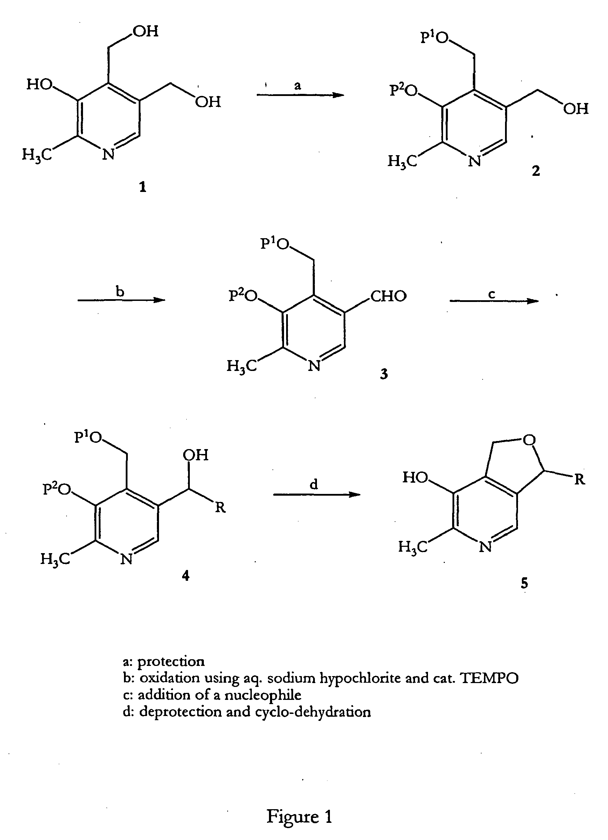

Figure 1 illustrates schematically the process of the present invention for the preparation of a 1,3-dihydro-6-methylfuro[3,4-c]pyridin-7-ol derivative 5 or a salt thereof. In a first step, the 3-hydroxy and 4-hydroxymethyl substituents of pyridoxine **1** or a salt thereof are protected to yield a 3,4-protected pyridoxine **2** or a salt thereof (step a in Figure 1).

Then the 5-hydroxymethyl substituent of the 3,4-protected pyridoxine **2** or the salt thereof is oxidised to the corresponding aldehyde using aqueous sodium hypochlorite in the presence of a catalytic amount of TEMPO (2,2,6,6-tetramethyl-1-piperidinyloxy) to yield a 3,4-protected pyridoxal **3** or a salt thereof (step b in Figure 1).

Instead of using unfavourable chromium or manganese compounds to effect the oxidation of a primary alcohol to an aldehyde, the process of the current invention uses aqueous sodium hypochlorite in the presence of a catalytic amount of TEMPO (2,2,6,6-tetramethyl-1-piperidinyloxy). The use of these reagents affords products, at ambient temperature, in excellent yield and the effluent contains sodium hypochlorite and TEMPO in only trace amounts.

In a third step, a nucleophile is added to the aldehyde of the 3,4-protected pyridoxal 3 or the salt thereof to yield an adduct **4** or a salt thereof (step c in Figure 1).

Then the adduct **4** or the salt thereof is deprotected and the 4- and 5-hydroxymethyl substituents of the deprotected adduct **4** or the salt thereof are cyclo-dehydrated to yield the desired 1,3-dihydro-6-methylfuro[3,4-c]pyridin-7-ol derivative **5** or a salt thereof (step d in Figure 1).

Figures 2 and 3 illustrate schematically preferred processes of the present invention.

Figure 2 shows a process for the preparation of 1,3-dihydro-6-methylfuro[3,4-c]pyridin-7-ol derivatives **5a**, wherein R is any atom or group and X is a leaving group such as a halide (e.g. Cl, Br or I).

Preferred embodiments of the invention are when R (in Figure 2) is an alkyl, an optionally substituted aryl or an optionally substituted heteroaryl group. A particularly preferred embodiment of the current invention is the preparation of cicletanine hydrochloride **5b** as described in Figure 3.

The sodium hypochlorite/TEMPO reagent combination has been used in the prior art (P.L. Anelli, F. Montanari and S. Quici, Organic Syntheses, coll. vol. 8, 367-371, 1993) for the oxidation of a primary alcohol to an aldehyde. However, to obtain a reasonable rate of reaction, it was reported that potassium bromide had to be added to the reaction mixture.

The inventors of the current invention have surprisingly shown that the oxidation reaction of primary alcohol **2a** to aldehyde **3a** is practically complete just after the addition of sodium hypochlorite (within 30-45 minutes) without the requirement of adding potassium bromide.

The inventors have found that the Grignard reaction to convert aldehyde **3a** to adduct **4b** does not go to completion, when the reaction conditions disclosed in patent US 4,383,998 were followed (i.e. addition of 3,4-isopropylidine pyridoxal **3a** at ~15-20°C). However, the inventors have found that the reaction could be driven to completion, when addition of 3,4-isopropylidine pyridoxal **3a** was done at elevated temperature (~70°C, THF reflux temperature). The yield was almost quantitative.

Similarly, the acid hydrolysis and cyclization of adduct **4b** to afford cicletanine hydrochloride **5b** did not proceed, when the reaction conditions reported in patent US 4,383,998 were followed (i.e. 12 hours at ambient temperature). The cyclization could only be achieved when the adduct **4b** was refluxed in concentrated HCl.

The process outlined in Figure 3 is an example of a procedure comprising the process of the current invention and detailed procedures for this process are found in the experimental section.

The process of the present invention is short and simple and avoids the use of disadvantageous reagents such as chromium trioxide and manganese dioxide. The process is easy to scale up for industrial scale manufacturing, and all raw materials and chemicals used in the process are environmentally friendly and do not require special treatment for disposal. Optionally 1 ,3-dihydro-6-methylfuro[3,4-c]pyridin-7-ol derivatives 5 and salts thereof may be manufactured in batches of 5kg or more, or even 10kg or more.

### Experimental procedure

### 3.4-Isopropylidine pyridoxine 2a

Preparation of this intermediate had been described in GB patent 1,286,161, but following the disclosed procedure the required intermediate could not be prepared. It was observed that the concentration of HCl was critical so as to get the desired ketal (3,4-isopropylidine). With lower concentration of HCl (4-8% w/w) the unwanted 4,5-isopropylidine pyridoxine hydrochloride (7-member ketal) had formed exclusively. To get the desired 6-member ketal (3,4-isopropylidine pyridoxine hydrochloride), higher concentration of HCl (not less than 15% w/w) was necessary. 8-15% w/w concentration of HCl afforded a mixture of 6- and 7-member ketal.

### Procedure:

Pyridoxine hydrochloride **1a** (200 g, 0.975 mole) was suspended in 4000 ml of preformed acetone/HCl solution* (~15-20% w/w HCl) at 20-30°C. The suspension was stirred at this temperature for 6 hours. After about 30-45 minutes the reaction mixture became almost clear (pale yellow colour) and then slowly precipitation started [formation of 3,4-isopropylidine hydrochloride (ketal hydrochloride)]. The stirring was continued for 6 hours. The progress of the reaction was monitored by HPLC. After stirring for 6 hours, the yellow-orange coloured reaction mixture was cooled to 0 to -5°C for 30 minutes and filtered. The wet cake was further washed with cold acetone (4 x 200 ml) and dried at 65-70°C under reduced pressure (~250 mm of Hg) to constant weight. The ketal hydrochloride was obtained in ~85% yield (204 g).
(* Dry HCl gas was dissolved in cooled acetone (0-10°C) till 15-20% w/w (typically 17-19% w/w) HCl concentration was obtained.)
M.P: 209-211°C [Lit. M.P: 210-212°C, W. Korytnyk and W. Wiedeman, J. Chem. Soc., 2531-2532 (1962)].
¹H-NMR (DMSO-d⁶): 1.56 ppm (6H, s, O-C(CH₃)₂-O); 2.55 ppm (3H, s, Ar-CH₃); 4.58 ppm (1H, s, -CH₂-O-); 5.08 ppm (2H, s, -CH₂-O-); 8.17ppm [1H, s, Ar-H (pyridine ring)].
Mass Spec: M⁺ (209), 151, 122, 94 and 81.

The free ketal **2a** was obtained by stirring the above ketal hydrochloride (204 g) in aqueous sodium carbonate [sodium carbonate 92.5 g, (1.05 equivalent) in 1020 ml of water] at room temperature (20-30°C) for 30 minutes. The ketal hydrochloride dissolved after charging and the ketal **2a** precipitated out immediately. The free ketal **2a** was isolated by filtration. The wet cake was washed with water (2 x 400 ml) followed by acetone washing (2 x 200 ml) and dried at 65-70°C under reduced pressure (~250 mm of Hg). The yield was 90% (157 g).
M.P: 108-110°C [Lit. M.P: 111-112°C, W. Korytnyk and W. Wiedeman, J. Chem. Soc., 2531-2532 (1962)].
¹H-NMR (DMSO-d⁶): 1.49 ppm (6H, s, O-C(CH₃)₂-O); 2.28 ppm (3H, s, Ar-CH₃); 4.41 ppm (1H, d, J= 5.3Hz, -OH); 4.48 ppm (2H, s, -CH₂-O-); 5.15 ppm (2H, t, J= 5.3Hz, -CH₂-OH); 7.92 ppm [1H, s, Ar-H (pyridine ring)].
Mass Spec: M⁺ (209), 151, 122, 94 and 81.

### 3.4-Isopropylidine pyridoxal 3a

To a suspension of 3,4-isopropylidine pyridoxine **2a** (157 g) and TEMPO (2,2,6,6-tetramethyl-1-piperidinyloxy) in 1570 ml of dichloromethane, aqueous sodium hypochlorite [1475 ml, (~4% w/w free chlorine available)] was added at 15-25°C over a period of 60 minutes. The pH of aqueous sodium hypochlorite was adjusted to ~9.5 with sodium hydrogen carbonate. The resulting biphasic reaction mixture was further stirred for one hour. By this time the initial suspension had turned into a clear biphasic mixture. The progress of the reaction was followed by HPLC. The conversion was complete within one hour after the complete addition of sodium hypochlorite. The two layers were separated and the aqueous layer was extracted with dichloromethane (4 x 390 ml). The combined organic layer was washed with 2% w/w aqueous sodium thiosulfate solution (4 x 680 ml) and with water (2 x 680 ml). The dichloromethane was then distilled under reduced pressure (~450-500 mm of Hg) at ~35-40°C to get a syrupy mass. This was swapped with cyclohexane (2 x 320 ml) to remove the traces of dichloromethane. The slurry thus obtained was then cooled to 0-5°C for 30 minutes and 3,4-isopropylidine pyridoxal **3a** was isolated by filtration. This was washed with cyclohexane (2 x 160ml) and then dried at 45°C under reduced pressure (~250 mm of Hg) till constant weight was obtained. The yield was 80% (125 g).
M.P: 58-60°C [Lit. M.P: 59-60°C, W. Korytnyk, E.J. Kris and R.P. Singh, J. Org. Chem., 29, 574-579 (1964)].
¹H-NMR (DMSO-d⁶): 1.51 ppm (6H, s, O-C(CH₃)₂-O); 2.40 ppm (3H, s, Ar-CH₃); 5.12 ppm (2H, s, -CH₂-O-); 8.57 ppm [1H, s, Ar-H (pyridine ring)]; 10.05 ppm (1H, s, -CHO).
Mass Spec: (207), 149, and 121, 80.

### 4-Chlorophenyl-5-[(3,4-isopropylidine)-2-methylpyridine]-methanol hydrochloride 4b

To a refluxing (~70°C) suspension of magnesium metal turning (18.23 g, 1.25 eq) in 500 ml of THF was added 4-bromochlorobenzene (14.45 g, 10% of total quantity) and a crystal of iodine. After the initiation of the reaction, the remaining quantity of 4-bromochlorobenzene (130.05 g in 200 ml of THF) was added over a period of 60 minutes at reflux temperature. 30 minutes after the complete addition, 125 g of 3,4-isopropylidine pyridoxal **3a** (in 200 ml of THF) was added at ~70°C [THF reflux temperature] to the reaction mixture (Grignard reagent prepared) over ~45 minutes. After the addition, refluxing was continued for 2 hours. Then THF was distilled off under reduced pressure [~600 mm of Hg, recovery of THF ~50-60% (v/v)] to get a thick residue. The residue obtained was cooled to ~40°C and was then quenched with 1 M HCl (1300 ml). The mixture was then cooled to 5-10°C for 30 minutes when 4-chlorophenyl-5-[(3,4-isopropylidine)-2-methylpyridine]-methanol hydrochloride **4b** precipitated as off-white granules. The product was isolated by filtration and was then washed with dichloromethane (2 x 260 ml). This was then dried at 70°C under reduced pressure (~250 mm of Hg) till constant weight. The yield was 85% (183 g).
M.P: 189-193°C.
¹H-NMR (DMSO-d⁶): 1.46 ppm (3H, s, O-C(CH₃)-O); 1.51 ppm (3H, s, O-C(CH₃)-O); 2.53 ppm (3H, s, Ar-CH₃); 4.84 ppm (2H, q, J= 17Hz, -CH₂-O-); 5.95 ppm (1H, bs, Ar-CH-O-); 7.39 ppm [2H, d, J= 8.4Hz, Ar-H (chlorophenyl ring)]; 7.45 [2H, d, J= 8.2Hz , Ar-H (chlorophenyl ring)]; 8.25 ppm [1H, s, Ar-H (pyridine ring)].
Mass Spec: (319), 262, 243, 226, 149, 139, and 77.

### 3-(4-Chlorophenyl)-1,3-dihydro-6-methylfuro[3,4-c]pyridin-7-ol hydrochloride 5b [Cicletanine hydrochloride]

100 g intermediate 4-chlorophenyl-5-[(3,4-isopropylidine)-2methylpyridine]-methanol hydrochloride **4b** was refluxed (95-100°C) in 300 ml of conc. HCl until the level of adduct **4b** decreased below 0.5%. The conversion was monitored by HPLC and this was achieved within 3 hours of reflux. As the reflux progressed, after about 30 minutes, the initial slurry changed into a clear solution. Then after about 2 hours the product (cicletanine hydrochloride **5b**) started falling out and finally slurry was obtained at the end of the conversion. This slurry was cooled to 0-5°C and the product was isolated by filtration. The wet cake was washed with chilled water (2 x 100 ml). This was dried at 70°C under reduced pressure (250 mm of Hg) till constant weight. The title compound **5b** was obtained as pale yellow powder in 75% yield (62.5 g).
M.P: 220-228°C [Lit. M.P: 219-228°C, US patent 4,383,998].
¹H-NMR (CD₃OD): 2.69 ppm (3H, s, Ar-CH₃); 4.45 ppm (2H, q (dd), J= 15.5 and 1.6Hz, -CH2-O-); 6.39 ppm (1H, bs, Ar-CH-O-); 7.45 ppm [4H, m, Ar-H (chlorophenyl ring)]; 7.95 ppm [1H, s, Ar-H (pyridine ring)].
¹H-NMR (CD₃OD + CDCl₃): 2.68 ppm (3H, s, Ar-CH₃); 5.40 ppm (2H, q (dd), J= 15.5 and 1.6Hz, -CH₂-O-); 6.33 ppm (1H, bs, Ar-CH-O-); 7.53 ppm [4H, m, Ar-H (chlorophenyl ring)]; 7.84 ppm [1H, s, Ar-H (pyridine ring)].

The above spectrum (CD₃OD +CDCl₃) was also in very good agreement with one reported in US patent 5,047,537 for (-) isomer of cicletanine. ¹H-NMR (CD₃OD + CDCl₃) reported in US 5,047,537 for (-) isomer of cicletanine: 2.46 ppm (3H, s, Ar-CH₃); 5.21 ppm (2H, q (dd), J= 15.5 and 1.6Hz, -CH₂-O-); 6.16 ppm (1H, bs, Ar-CH-O-); 7.31 ppm [4H, m, Ar-H (chlorophenyl ring)]; 7.56 ppm [1H, s, Ar-H (pyridine ring)].
¹³C-NMR (CD₃OD): 15.40 ppm [Ar-CH₃ (pyridine ring)], 73.22 ppm (-O-CH₂), 85.86 ppm (Ar-CH-O-); 127.51 (Ar-C); 130.39 (2 x Ar-C phenyl ring); 130.89 (2 x Ar-C phenyl ring); 136.62 (Ar-C); 140.73 (Ar-C); 143.65 (Ar-C); 144.91 (2 x Ar-C pyridine ring C2); 150.97 (Ar-C pyridine ring C6).
Mass: M⁺-HCl (262), 226, 139.

This was later treated with activated carbon in water and pure cicletanine hydrochloride **5b** was isolated as off-white material in 75% yield.

## Claims

1. A process for the preparation of a 1,3-dihydro-6-methylfuro[3,4-c]pyridin-7-ol derivative 5 or a salt thereof, comprising the steps of:
(a) protecting the 3-hydroxy and 4-hydroxymethyl substituents of pyridoxine **1** 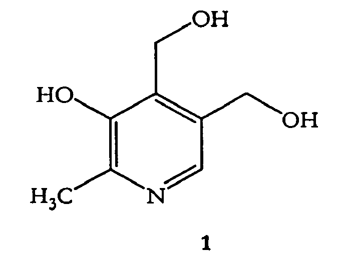 or a salt thereof, to yield a 3,4-protected pyridoxine **2** 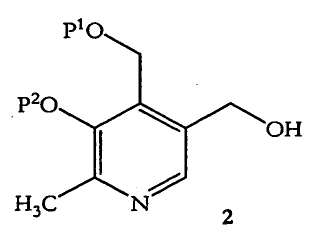 or a salt thereof, wherein P¹ and P² are independently protecting groups or together form one protecting group,
(b) oxidising the 5-hydroxymethyl substituent of the 3,4-protected pyridoxine 2 or the salt thereof, using aqueous sodium hypochlorite in the presence of a catalytic amount of TEMPO (2,2,6,6-tetramethyl-1-piperidinyloxy) to yield a 3,4-protected pyridoxal **3** 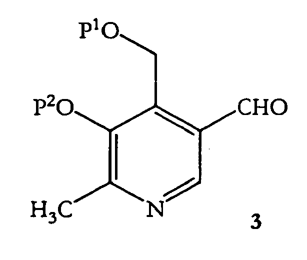 or a salt thereof,
(c) adding a nucleophile to the aldehyde of the 3,4-protected pyridoxal **3** or the salt thereof to yield an adduct **4** 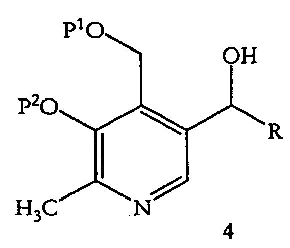 or a salt thereof, wherein R is any atom or group, and
(d) deprotecting the adduct **4** or the salt thereof, and cyclo-dehydrating the 4- and 5-hydroxymethyl substituents of the deprotected adduct **4** or the salt thereof to yield the 1,3-dihydro-6-methylfuro[3,4-c]pyridin-7-ol derivative **5** 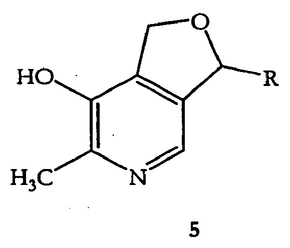 or a salt thereof.

2. A process as claimed in claim 1, wherein R is an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton.

3. A process as claimed in claim 1 or claim 2, wherein protecting groups P¹ and P² are independently -R³, -Si(R³)₃, -COR³, -CO₂R³, -SO₃⁻ or -SO₃R³, wherein each R³ is independently an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, or wherein protecting groups P¹ and P² together form one protecting group -CHR⁴-, -C(R⁴)₂-, -C(OR⁴)R⁵-, -C(N(R⁴)₂)R⁵-, -Si(R⁴)₂-, -Si(R⁴)₂-O-Si(R⁴)₂-, -CO- or -BR⁴-, wherein each R⁴ and each R⁵ is independently an optionally substituted alkyl, alkenyl, alkynyl, aryl, arylalkyl, arylalkenyl, arylalkynyl, alkylaryl, alkenylaryl or alkynylaryl group, which may include one or more heteroatoms N, O or S in its carbon skeleton, or wherein R⁴ and R⁴, or R⁴ and R⁵, together form an optionally substituted cycloalkyl or cycloheteroalkyl group.

4. A process as claimed in claim 3, wherein protecting groups P¹ and P² together form one protecting group.

5. A process as claimed in any preceding claim, wherein the nucleophile used in step (c) is:
(a) a Grignard reagent RMgX, wherein R is any atom or group, and wherein X is a leaving group;
(b) Li-R, wherein R is any atom or group;
(c) Zn-R, wherein R is an optionally substituted alkyl group;
(d) Na-C≡C-Rⁿ, wherein Rⁿ is any atom or group, and wherein R in formulae **4, 4a, 5** and **5a** is -C≡C-Rⁿ;
(e) R'₂Al-CH=CHRⁿ, wherein Rⁿ is any atom or group, wherein each R' is independently an alkyl group, and wherein R in formulae **4, 4a, 5** and **5a** is -CH=CHRⁿ;
(f) R'₃Sn-CH₂-CH=CHRⁿ, wherein Rⁿ is any atom or group, wherein each R' is independently an alkyl group, and wherein R in formulae **4, 4a, 5** and **5a** is -CH₂-CH=CHRⁿ;
(g) R₂CuLi, wherein R is an unsubstituted alkyl group; or
(h) (R'O)₃TiR, (R'₂N)₃TiR or (R'O)₃ZrR, wherein R is any atom or group, and wherein each R' is independently an unsubstituted alkyl group.

6. A process as claimed in claim 5, wherein the nucleophile used in step (c) is:
(a) a Grignard reagent RMgX which is prepared *in situ* from magnesium and R-X, wherein R and X are defined as in claim 5(a);
(b) Li-R which is prepared *in situ* from lithium and R-X, wherein R is defined as in claim 5(b) and X is a leaving group; or
(c) -(R'O)₃TiR, (R'₂N)₃TiR or (R'O)₃ZrR which is prepared by transmetallation of a Grignard reagent RMgX or Li-R with (R'O)₃TiX, (R'₂N)₃TiX or (R'O)₃ZrX, wherein R is defined as in claim 5(h) and X is independently a halide.

7. A process as claimed in claim 5 or claim 6, wherein the nucleophile used in step (c) is a Grignard reagent RMgX, and wherein the addition of the Grignard reagent RMgX to the aldehyde of the 3,4-protected pyridoxal **3** or the salt thereof is carried out at a temperature of 50 to 130°C.

8. A process as claimed in any preceding claim, wherein the oxidation of step (b) is carried out in the presence of less than 3 mol% KBr compared to the alcohol 2 to be oxidised.

9. A process as claimed in claim 8, wherein the oxidation of step (b) is carried out substantially in the absence of KBr.

10. A process as claimed in any preceding claim, wherein the deprotection and cyclo-dehydration of step (d) is achieved in one step by heating in HCl, yielding a 1,3-dihydro-6-methylfuro[3,4-c]pyridin-7-ol derivative hydrochloric acid salt **5a**.

## Patentansprüche

1. Verfahren für die Zubereitung eines 1,3-Dihydro-6-methylfuro[3,4-c]pyridin-7-ol-Derivats 5 oder eines Salzes desselben, umfassend die Schritte des:
(a) Schützens des 3-Hydroxy- und 4-Hydroxymethylsubstituenten von Pyridoxin 1 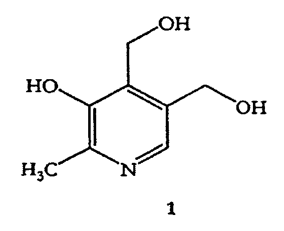 oder eines Salzes desselben unter Bildung eines 3,4-geschütztem Pyridoxins 2 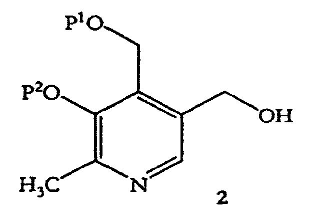 oder eines Salzes desselben, wobei P¹ und P² unabhängige Schutzgruppen sind oder zusammen eine Schutzgruppe bilden,
(b) Oxidieren des 5-Hydroxymethylsubstituenten des 3,4-geschützten Pyridoxins 2 oder des Salzes desselben mit Hilfe von wässrigem Natriumhypochlorit in Gegenwart einer katalytischen Menge von TEMPO (2,2,6,6-Tetramethyl-1-piperidinyloxy) unter Bildung eines 3,4-geschützten Pyridoxals 3 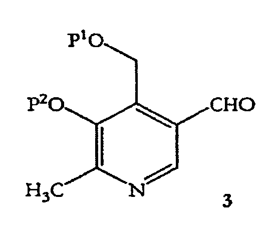 oder eines Salzes desselben,
(c) Zugeben eines Nukleophils zum Aldehyd des 3,4-geschützten Pyridoxals 3 oder des Salzes desselben unter Bildung eines Addukts 4 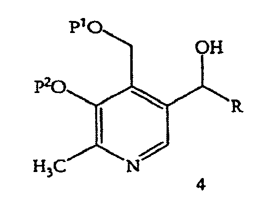 oder eines Salzes desselben, wobei R irgendein Atom oder irgendeine Gruppe ist, und
(d) Entschützen des Addukts 4 oder des Salzes desselben und Cyclodehydratisieren der 4- und 5-Hydroxymethylsubstituenten des entschützten Addukts 4 oder des Salzes desselben unter Bildung des 1,3-Dihydro-6-methylfuro[3,4-c]pyridin-7-ol-Derivats 5 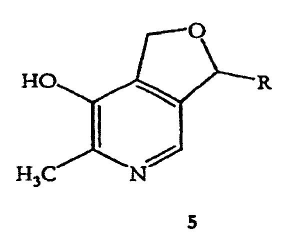 oder des Salzes desselben.

2. Verfahren nach Anspruch 1, wobei R eine wahlweise substituierte Alkyl-, Alkenyl-, Alkynyl-, Aryl-, Arylalkyl-, Arylalkenyl-, Arylalkynyl-, Alkylaryl-, Alkenylaryl- oder Alkynylarylgruppe ist, die ein oder mehrere Heteroatome N, O oder S in ihrem Kohlenstoffskelett enthalten können.

3. Verfahren nach Anspruch 1 oder Anspruch 2, wobei die Schutzgruppen P¹ und P² unabhängig für -R³, -Si(R³)₃, -COR³, -CO₂R³, -SO₃⁻ oder SO₃R³ stehen, wobei jedes R³ unabhängig eine wahlweise substituierte Alkyl-, Alkenyl-, Alkynyl-, Aryl-, Arylalkyl-, Aryalkenyl-, Arylalkynyl-, Alkylaryl-, Alkenylaryl- oder Alkynylarylgruppe ist, die ein oder mehrere Heteroatome N, O oder S in ihrem Kohlenstoffskelett enthalten können oder wobei die Schutzgruppen P¹ und P² zusammen eine Schutzgruppe -CHR⁴-, -C(R⁴)₂-, -C(OR⁴)R⁵-, -C(N(R⁴)₂)R⁵-, -Si(R⁴)₂-, -Si(R⁴)₂-O-Si(R⁴)₂-, -CO- oder -BR⁴- bilden, wobei jedes R⁴ und jedes R⁵ unabhängig eine wahlweise substituierte Alkyl-, Alkenyl-, Alkynyl-, Aryl-, Arylalkyl-, Aryalkenyl-, Arylalkynyl-, Alkylaryl-, Alkenylaryl- oder Alkynylarylgruppe ist, die ein oder mehrere Heteroatome N, O oder S in ihrem Kohlenstoffskelett enthalten können, oder wobei R⁴ und R⁴ oder R⁴ und R⁵ zusammen eine wahlweise substituierte Cycloalkyl- oder Cycloheteroalkylgruppe bilden.

4. Verfahren nach Anspruch 3, wobei die Schutzgruppen P¹ und P² zusammen eine Schutzgruppe bilden.

5. Verfahren nach einem der vorhergehenden Ansprüche, wobei das in Schritt (c) verwendete Nukleophil folgendes ist:
(a) ein Grignard-Reagens RMgX, wobei R irgendein Atom oder irgendeine Gruppe ist und wobei X eine Austrittsgruppe ist;
(b) Li-R, wobei R irgendein Atom oder irgendeine Gruppe ist;
(c) Zn-R, wobei R eine wahlweis substituierte Alkylgruppe ist;
(d) Na-C =C-Rⁿ, wobei Rⁿ irgendein Atom oder irgendeine Gruppe ist und wobei jedes R in den Formeln 4, 4a, 5 und 5a -C ≡C-Rⁿ ist;
(e) R'₂Al-CH=CHRⁿ wobei Rⁿ irgendein Atom oder irgendeine Gruppe ist, wobei jedes R' unabhängig eine Alkylgruppe ist und wobei R in Formeln 4, 4a, 5 und 5a -CH=CHRⁿ ist;
(f) R'₃Sn-CH₂-CH=CHRⁿ, wobei Rⁿ irgendein Atom oder irgendeine Gruppe ist, wobei jedes R' unabhängig eine Alkylgruppe ist und wobei R in Formeln 4, 4a, 5 und 5a -CH₂-CH=CHRⁿ ist;
(g) R₂CuLi, wobei R eine unsubstituierte Alkylgruppe ist; oder
(h) (R'O)₃TiR, (R'₂N)₃TiR oder (R'O)₃ZrR, wobei R irgendein Atom oder irgendeine Gruppe ist und wobei jedes R' unabhängig eine unsubstituierte Alkylgruppe ist.

6. Verfahren nach Anspruch 5, wobei das in Schritt (c) verwendete Nukleophil folgendes ist:
(a) ein Grignard-Reagens RMgX, das in situ aus Magnesium und R-X zubereitet wird, wobei R und X die in Anspruch 5(a) angegebene Definition aufweisen;
(b) Li-R, das in situ aus Lithium und R-X zubereitet wird, wobei R die in Anspruch 5(a) angegebene Definition aufweist und X eine Austrittsgruppe ist; oder
(c) (R'O)₃TiR, (R'₂N)₃TiR oder (R'O)₃ZrR, das durch Transmetallisation eines Grignard-Reagens RMgX oder Li-R mit (R'O)₃TiX, (R'₂N)₃TiX oder (R'O)₃ZrX zubereitet wird, wobei R die in Anspruch 5(h) angegebene Definition aufweist und X unabhängig ein Halogenid, ist.

7. Verfahren nach Anspruch 5 oder 6, wobei das in Schritt (c) verwendete Nukleophil ein Grignard-Reagens RMgX ist und wobei der Zusatz des Grignard-Reagens RMgX zu dem Aldehyd des 3,4-geschützten Pyridoxals 3 oder des Salzes desselben bei einer Temperatur von 50 bis 130°C durchgeführt wird.

8. Verfahren nach einem der vorhergehenden Ansprüche, wobei die Oxidation von Schritt (b) in Gegenwart von weniger als 3 Mol-% KBr, im Vergleich mit dem zu oxidierenden Alkohol 2, durchgeführt wird.

9. Verfahren nach Anspruch 8, wobei die Oxidation von Schritt (b) im Wesentlichen in Abwesenheit von KBr durchgeführt wird.

10. Verfahren nach einem der vorhergehenden Ansprüche, wobei die Entschützungs- und Cyclodehydratisierung von Schritt (d) in einem Schritt durch Erhitzen in HCl erreicht wird unter Bildung von 1,3-Dihydro-6-methylfuro[3,4-c]pyridin-7-ol-Derivat-Salzsäuresalz 5a.

## Revendications

1. Procédé de préparation d'un dérivé 5 de 1,3-dihydro-6-méthylfuro[3,4-c]pyridin-7-ol ou d'un sel de celui-ci, comprenant les étapes consistant à :
(a) protéger les substituants 3-hydroxy et 4-hydroxyméthyle d'une pyridoxine 1 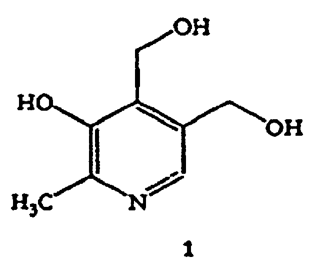 ou d'un sel de celle-ci, pour donner une pyridoxine 2 3,4-protégée 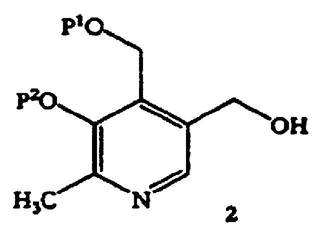 ou un sel de celle-ci, dans laquelle P¹ et P² sont indépendamment des groupes protecteurs ou forment ensemble un groupe protecteur,
(b) oxyder le substituant 5-hydroxyméthyle de la pyridoxine 2 3,4-protégée ou le sel de celle-ci, en utilisant de l'hypochlorite de sodium aqueux en présence d'une quantité catalytique de TEMPO (2,2,6,6-tétraméthyl-1-pipéridinyloxy) pour donner un pyridoxal 3 3,4 protégé 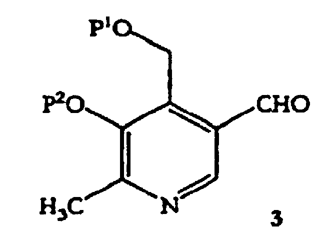 ou un sel de celui-ci,
(c) ajouter un nucléophile à l'aldéhyde du pyridoxal 3 3,4-protégé ou du sel de celui-ci pour donner un produit d'addition 4 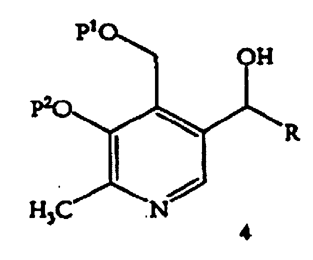 ou un sel de celui-ci, dans lequel R est tout atome ou groupe, et
(d) déprotéger le produit d'addition 4 ou le sel de celui-ci, et cyclo-déshydrater les substituants 4- et 5-hydroxyméthyle du produit d'addition 4 déprotégé ou du sel de celui-ci pour donner le dérivé 5 de 1,3-dihydro-6-méthylfuro[3,4-c]pyridin-7-ol 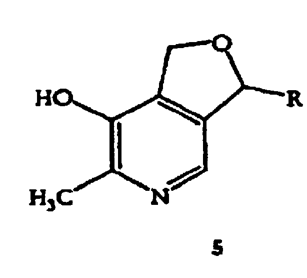 ou un sel de celui-ci.

2. Procédé selon la revendication 1, dans lequel R est un groupe alkyle, alcényle, alcynyle, aryle, arylalkyle, arylalcényle, arylalcynyle, alkylaryle, alcénylaryle ou alcynylaryle facultativement substitué, qui peut comprendre un ou plusieurs hétéroatomes N, O ou S dans son squelette carboné.

3. Procédé selon la revendication 1 ou la revendication 2, dans lequel les groupes protecteurs P¹ et P² sont indépendamment -R³, -Si(R³)₃, -COR³, -CO₂R³, -SO₃⁻ ou -SO₃R³, où chaque R³ est indépendamment un groupe alkyle, alcényle, alcynyle, aryle, arylalkyle, arylalcényle, arylalcynyle, alkylaryle, alcénylaryle ou alcynylaryle facultativement substitué, qui peut comprendre un ou plusieurs hétéroatomes N, O ou S dans son squelette carboné, ou dans lequel les groupes protecteurs P¹ et P² forment ensemble un groupe protecteur -CHR⁴-, -C(R⁴)₂-, - C(OR⁴)R⁵-, -C(N(R⁴)₂)R⁵-, -Si(R⁴)₂-, -Si(R⁴)₂-O-Si(R⁴)₂-, -CO- ou -BR⁴-, où chaque R⁴ et chaque R⁵ est indépendamment un groupe alkyle, alcényle, alcynyle, aryle, arylalkyle, arylalcényle, arylalcynyle, alkylaryle, alcénylaryle ou alcynylaryle facultativement substitué, qui peut comprendre un ou plusieurs hétéroatomes N, O ou S dans son squelette carboné, ou dans lequel R⁴ et R⁴, ou R⁴ et R⁵, forment ensemble un groupe cycloalkyle ou cyclohétéroalkyle facultativement substitué.

4. Procédé selon la revendication 3, dans lequel les groupes protecteurs P¹ et P² forment ensemble un groupe protecteur.

5. Procédé selon l'une quelconque des revendications précédentes, dans lequel le nucléophile utilisé dans l'étape (c) est :
(a) un réactif de Grignard RMgX, dans lequel R est tout atome ou groupe, et dans lequel X est un groupe partant ;
(b) Li-R, dans lequel R est tout atome ou groupe ;
(c) Zn-R, dans lequel R est un groupe alkyle facultativement substitué ;
(d) Na-C≡C-Rⁿ, dans lequel Rⁿ est tout atome ou groupe, et dans lequel R dans les formules 4, 4a, 5 et 5a est -C≡C-Rⁿ;
(e) R'₂Al-CH=CHRⁿ, dans lequel Rⁿ est tout atome ou groupe, dans lequel chaque R' est indépendamment un groupe alkyle, et dans lequel R dans les formules 4, 4a, 5 et 5a est -CH=CHRⁿ ;
(f) R'₃Sn-CH₂-CH=CHRⁿ, dans lequel Rⁿ est tout atome ou groupe, dans lequel chaque R' est indépendamment un groupe alkyle, et dans lequel R dans les formules 4, 4a, 5 et 5a est -CH₂-CH=CHRⁿ;
(g) R₂CuLi, dans lequel R est un groupe alkyle non substitué ; ou
(h) (R'O)₃TiR, (R'₂N)₃TiR ou (R'O)₃ZrR, dans lequel R est tout atome ou groupe, et dans lequel chaque R' est indépendamment un groupe alkyle non substitué.

6. Procédé selon la revendication 5, dans lequel le nucléophile utilisé dans l'étape (c) est :
(a) un réactif de Grignard RMgX qui est préparé *in situ* à partir de magnésium et de R-X, dans lequel R et X sont tels que définis dans la revendication 5(a) ;
(b) Li-R qui est préparé *in situ* à partir de lithium et de R-X, dans lequel R est défini comme dans la revendication 5(b) et X est un groupe partant ; ou
(c) (R'O)₃TiR, (R'₂N)₃TiR ou (R'O)₃ZrR qui est préparé par transmetallation d'un réactif de Grignard RMgX ou Li-R avec (R'O)₃TiX, (R'₂N)₃TiX ou (R'O)₃ZrX, dans lesquels R est défini comme dans la revendication 5(b) et X est indépendamment un halogénure.

7. Procédé selon la revendication 5 ou la revendication 6, dans lequel le nucléophile utilisé dans l'étape (c) est un réactif de Grignard RMgX, et dans lequel l'addition du réactif de Grignard RMgX à l'aldéhyde du pyridoxal 3 3,4-protégé ou du sel de celui-ci est réalisée à une température de 50 à 130 °C.

8. Procédé selon l'une quelconque des revendications précédentes, dans lequel l'oxydation de l'étape (b) est réalisée en présence de moins de 3 % en mol de KBr comparé à l'alcool 2 à oxyder.

9. Procédé selon la revendication 8, dans lequel l'oxydation de l'étape (b) est réalisée sensiblement en l'absence de KBr.

10. Procédé selon l'une quelconque des revendications précédentes, dans lequel la déprotection et la cyclo-déshydratation de l'étape (d) sont atteintes en une étape par chauffage dans du HCl, en donnant un sel d'acide chlorhydrique de dérivé 5a de 1,3-dihydro-6-méthylfuro[3,4-c]pyridin-7-ol.
